(19) Europäisches Patentamt
European Patent Office
Office européen des brevets

(11) EP 3 061 455 B1

(12) **EUROPEAN PATENT SPECIFICATION**

(45) Date of publication and mention of the grant of the patent:
**28.11.2018 Bulletin 2018/48**

(21) Application number: **14855333.2**

(22) Date of filing: **17.10.2014**

(51) Int Cl.:
***A61K 31/519*** *(2006.01)* ***A61K 9/00*** *(2006.01)*
***A61K 9/08*** *(2006.01)* ***A61P 27/02*** *(2006.01)*
***A61P 27/06*** *(2006.01)* ***A61P 43/00*** *(2006.01)*

(86) International application number:
**PCT/JP2014/077651**

(87) International publication number:
**WO 2015/060208 (30.04.2015 Gazette 2015/17)**

(54) **THERAPEUTIC AGENT FOR OCULAR DISEASE OR PROPHYLACTIC AGENT FOR OCULAR DISEASE**

THERAPEUTIKUM FÜR AUGENERKRANKUNG ODER PROPHYLAKTIKUM FÜR AUGENERKRANKUNG

AGENT THÉRAPEUTIQUE POUR MALADIE OCULAIRE OU AGENT PROPHYLACTIQUE POUR MALADIE OCULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **21.10.2013 JP 2013218352**

(43) Date of publication of application:
**31.08.2016 Bulletin 2016/35**

(73) Proprietor: **Japan Tobacco, Inc.**
**Tokyo 105-8422 (JP)**

(72) Inventors:
- **OKIGAMI, Hiromi**
  **Ikoma-shi**
  **Nara 630-0101 (JP)**
- **KUROSE, Tatsuji**
  **Ikoma-shi**
  **Nara 630-0101 (JP)**
- **FUJISAWA, Koushi**
  **Ikoma-shi**
  **Nara 630-0101 (JP)**
- **KIKKAWA, Chinami**
  **Ikoma-shi**
  **Nara 630-0101 (JP)**

(74) Representative: **Vossius & Partner**
**Patentanwälte Rechtsanwälte mbB**
**Siebertstrasse 3**
**81675 München (DE)**

(56) References cited:
**JP-A- 2011 046 700** **JP-A- 2012 176 930**
**JP-A- 2013 500 977** **US-A1- 2009 082 470**
**US-A1- 2011 136 778**

- **AL-LATAYFEH M ET AL: "Antiangiogenic Therapy for Ischemic Retinopathies", COLD SPRING HARBOR PERSPECTIVES IN MEDICINE, vol. 2, no. 6, 28 February 2012 (2012-02-28), pages a006411-a006411, XP055348507, DOI: 10.1101/cshperspect.a006411**
- **KIMOTO KENICHI ET AL: "Anti-VEGF Agents for Ocular Angiogenesis and Vascular Permeability", JOURNAL OF OPHTHALMOLOGY, vol. 19, no. 14, January 2012 (2012-01), pages 5731-11, XP055141705, ISSN: 2090-004X, DOI: 10.1001/archophthalmol.2008.1**
- **KANAKO IZUMI-NAGAI ET AL.: 'Interleukin-6 Receptor-Mediated Activation of Signal Transducer and Activator of Transcription-3 (STAT3) Promotes Choroidal Neovascularization' THE AMERICAN JOURNAL OF PATHOLOGY vol. 170, no. ISSUE, 2007, pages 2149 - 2158, XP055339393**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- HAIBO WANG ET AL.: 'VEGF-Mediated STAT3 Activation Inhibits Retinal Vascularization by Down-Regulating Local Erythropoietin Expression' THE AMERICAN JOURNAL OF PATHOLOGY vol. 180, no. ISSUE, 2012, pages 1243 - 1253, XP055237244

## Description

TECHNICAL FIELD

The present invention relates to novel medical use of 3-[(3S,4R)-3-Methyl-6-(7H-pyrrolo[2,3-d]pyrimidin-4-yl)-1,6 -diazaspiro[3.4]octan-1-yl]-3-oxopropanenitrile (hereinafter, referred to as Compound A). More specifically, the present invention relates to Compound A or a pharmaceutically acceptable salt thereof for use in the treatment or prevention of an eye disease selected from the group consisting of age-related macular degeneration, diabetic retinopathy, macular edema, retinal vein occlusion, neovascular maculopathy and neovascular glaucoma.

BACKGROUND ART

Compound A is described in Patent Literature 1. However, Compound A has not been known to inhibit functions of vascular endothelial growth factor (hereinafter, abbreviated as VEGF).

Compound A is represented by the following chemical structural formula:

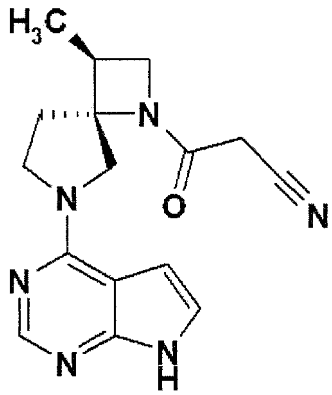

Compound A can be produced according to the method described in Patent Literature 1.

VEGF is a subfamily of growth factors that promote vasculogenesis (the de novo formation of blood vessels from endothelial cell precursors by differentiation) and angiogenesis (the formation of blood vessels from pre-existent vasculature). Also, VEGF is known to increase vascular permeability and induce dropsy.

An increase of VEGF gene expression is known to be involved in an increase of the vascular permeability and dropsy (Non-Patent Literature 1) and angiogenesis (Non-Patent Literature 2), which are characteristics of vascular diseases such as exudative age-related macular degeneration, diabetic retinopathy and central retinal vein occlusion.

An animal model in which the retinal vascular permeability is increased by intravitreal VEGF injection is known, and this model is widely used to evaluate candidate drugs for diabetic retinopathy, diabetic macular edema, retinal vein occlusion and so on (Non-Patent Literatures 3 and 4).

Ranibizumab (general name) is a drug approved for the manufacturing and sale of pharmaceuticals, etc. under the provision of Article 14 of the Japanese Pharmaceutical Affairs Law (approval number: 22100AMX00399000). Ranibizumab, which is a Fab fragment of humanized mouse anti-human VEGF monoclonal antibody, is known to be effective for treatment of subfoveal choroidal neovascularization secondary to age-related macular degeneration, macular edema following retinal vein occlusion and choroidal neovascularization secondary to pathologic myopia.

Aflibercept (general name) is a drug approved for the manufacturing and sale of pharmaceuticals, etc. under the provision of Article 14 of the Japanese Pharmaceutical Affairs Law (approval number: 22400AMX01389). Aflibercept is a recombinant fusion glycoprotein in which the extracellular domains of human VEGF receptors 1 and 2 are fused to the Fc domain of human IgG1. Aflibercept is known to be effective for treatment of subfoveal choroidal neovascularization secondary to age-related macular degeneration since Aflibercept binds to VEGF-A and PIGF as a soluble decoy receptor to inhibit the actions of the same.

Pegaptanib (general name) is a drug approved for the manufacturing and sale of pharmaceuticals, etc. under the provision of Article 14 of the Japanese Pharmaceutical Affairs Law (approval number: 22000AMX01705). Pegaptanib is a pegylated oligonucleotide that selectively binds to VEGF-$A_{165}$ with high affinity to inhibit the activity of VEGF-$A_{165}$, and is known to be effective for treatment of subfoveal choroidal neovascularization secondary to age-related macular degeneration.

The above description suggests that inhibiting the functions of VEGF to suppress an increase of the retinal vascular permeability and neovascularization from choroid is effective for treatment or prevention of eye diseases such

as age-related macular degeneration, diabetic retinopathy, macular edema, retinal vein occlusion, neovascular maculopathy and neovascular glaucoma.

Age-related macular degeneration is a disease occurring in the macular area with age. Age-related macular degeneration is classified into the exudative form and atrophic form depending on the presence or absence of choroidal neovascularization. Exudative age-related macular degeneration is also called neovascular macular degeneration, and is characterized by occurrence of edema in the macula caused by bleeding or exuding from neovascular vessels originating from the choroid extending in the macular area. Atrophic age-related macular degeneration does not involve choroidal neovascularization, and is characterized by atrophy of retinal pigment epithelial cells and choriocapillaris to result in disorder of the retina.

Diabetic retinopathy is one of the three major complications of diabetes, and nowadays ranks the highest causes of adults' loss of vision. Diabetic retinopathy is a retinal vascular disease, and starts as microangiopathy in the capillary level. An initial lesion of angiopathy is called simple diabetic retinopathy, and the condition that occlusion of capillary is advanced as a result of progression of the lesion is called nonproliferative diabetic retinopathy, and the condition that occlusion of vessels extends and retinal ischemia progresses, resulting in neovascularization in the retina and vitreous body is called proliferative diabetic retinopathy.

Macular edema refers to the condition that edema occurs in the macula which is part of the retina. Diabetic macular edema occurs in any stages of diabetic retinopathy from simple diabetic retinopathy to proliferative diabetic retinopathy, and causes decreased vision.

Retinal vein occlusion is retinal vascular occlusion caused by hypertension, arterial sclerosis and so on. When a retinal vein is occluded at its root, it is called central retinal vein occlusion and bleeding occurs all over the retina. When a branch of a retinal vein is occluded, it is called branch retinal vein occlusion, and when a temporal vein of the center nerve is occluded, macular edema that causes visual impairment develops.

Neovascular maculopathy is a disease causing an irreversible decrease in vision due to breakdown of the structure of the macular area by neovascular vessels.

Neovascular glaucoma is a disease developed when diabetic retinopathy advances to proliferative diabetic retinopathy, and neovascular vessels arise in the iris and the iridocorneal angle, and fibrovascular proliferative membrane occludes the iridocorneal angle which is the outlet of aqueous humor to increase the intraocular pressure.

## CITATION LISTS

### PATENT LITERATURE

Patent Literature 1: JP 2011-46700 A.

### NON-PATENT LITERATURES

Non-Patent Literature 1: CRAWFORD, Y et al. VEGF inhibition: insights from preclinical and clinical studies. Cell Tissue Res. Jan 2009, Vol.335, No.1, pages 261-269.

Non-Patent Literature 2: DVORAK, HF et al. Vascular permeability factor/vascular endothelial growth factor and the significance of microvascular hyperpermeability in angiogenesis. Curr Top Microbiol Immunol. 1999, Vol.237, pages 97-132.

Non-Patent Literature 3: XU, Q et al. Sensitive blood-retinal barrier breakdown quantitation using Evans blue. Invest Ophthalmol Vis Sci. Mar 2001, Vol.42, No.3, pages 789-794.

Non-Patent Literature 4: EDELMAN, JL et al. Corticosteroids inhibit VEGF-induced vascular leakage in a rabbit model of blood-retinal and blood-aqueous barrier breakdown. Exp Eye Res. Feb 2005, Vol. 80, No.2, pages 249-258.

## SUMMARY OF INVENTION

### Technical Problem

The problem to be solved by the invention is to provide novel medical use of Compound A.

As a result of diligent efforts for developing novel medical use of Compound A, the present inventors have found for the first time that Compound A suppresses an increase of the retinal vascular permeability induced by VEGF; that Compound A is effective for various eye diseases involving VEGF owing to the aforementioned suppression; and that Compound A passes through the blood-retina barrier, and accomplished the present invention.

The present invention includes the following embodiments.

[1] An agent for use in the treatment or prevention of an eye disease selected from the group consisting of age-related macular degeneration, diabetic retinopathy, macular edema, retinal vein occlusion, neovascular maculopathy and neovascular glaucoma, comprising as an active ingredient a compound represented by the following chemical structural formula:

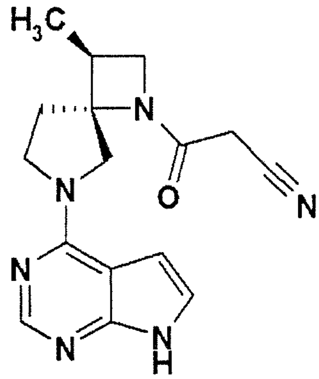

or a pharmaceutically acceptable salt thereof.

[2] The agent for use according to [1], wherein the age-related macular degeneration is exudative age-related macular degeneration.

[3] The agent for use according to [1], wherein the macular edema is diabetic macular edema or macular edema following branch retinal vein occlusion.

[4] The agent for use according to [1], wherein the retinal vein occlusion is central retinal vein occlusion.

[5] The agent for use according to [1], wherein the therapeutic or preventive agent is orally administered.

[6] The agent for use according to [1], wherein the therapeutic or preventive agent is administered to the eyes.

[7] The agent for use according to [6], wherein an administration site is a vitreous body.

[8] The agent for use according to [6] or [7], wherein a dosage form is an injection.

The invention also relates to a pharmaceutical composition for use in the treatment or prevention of an eye disease selected from the group consisting of age-related macular degeneration, diabetic retinopathy, macular edema, retinal vein occlusion, neovascular maculopathy and neovascular glaucoma, comprising Compound A or a pharmaceutically acceptable salt thereof as active ingredient.

Advantageous Effects of Invention

Compound A is effective as a therapeutic or preventive agent for various eye diseases involving VEGF because Compound A suppresses an increase of the retinal vascular permeability induced by VEGF.

BRIEF DESCRIPTION OF DRAWINGS

Fig. 1 shows a chart illustrating the suppression of retinal vascular permeability by oral administration of Compound A in a rat model where the VEGF-induced retinal vascular permeability was increased. (Example 1)

Fig. 2 shows a chart illustrating the suppression of retinal vascular permeability by intravitreal injection of Compound A in a rat model where the VEGF-induced retinal vascular permeability was increased. (Example 2)

Description of Embodiments

Terms and phrases used herein are defined as below.

The "pharmaceutically acceptable salt" may be any salt as long as it forms an non-toxic salt with Compound A, and includes a salt with an inorganic acid, a salt with an organic acid, a salt with an amino acid, etc.

The salt with an inorganic acid includes a salt with hydrochloric acid, nitric acid, sulfuric acid, phosphoric acid, hydrobromic acid, etc.

The salt with an organic acid includes a salt with oxalic acid, maleic acid, citric acid, fumaric acid, lactic acid, malic acid, succinic acid, tartaric acid, acetic acid, trifluoroacetic acid, gluconic acid, ascorbic acid, methanesulfonic

acid, benzenesulfonic acid, p-toluenesulfonic acid, etc.

The salt with an amino acid includes a salt with aspartic acid, glutamic acid, etc.

According to known methods, each salt may be obtained by reacting Compound A with an inorganic acid, an organic acid or an amino acid.

The "pharmaceutical composition" refers to a composition useable as a pharmaceutical.

The pharmaceutical composition for use according to the present invention is produced by appropriately mixing Compound A or a pharmaceutically acceptable salt thereof with at least one or more types of pharmaceutically acceptable carriers and the like in appropriate amounts according to a method known in the technical field of pharmaceutical preparation. The content of Compound A or a pharmaceutically acceptable salt thereof in the pharmaceutical composition differs depending on its dosage form, dosage amount, etc.

The "pharmaceutical composition" can be orally or parenterally administered. The administration mode includes oral administration, ophthalmic local administration (such as instillation, administration into conjunctival sac, intravitreal injection, subconjunctival injection, and injection into Tenon's capsule), intravenous administration, percutaneous administration, etc. The dosage form suited for oral administration includes a tablet, a capsule, a granule, a powder, a lozenge, a syrup, an emulsion, a suspension, etc., and the dosage form suited for parenteral administration includes an external preparation, a suppository, an injection, an eye drop, an eye ointment, a patch, a gel, an insert, a nasal drug, a pulmonary drug, etc. These can be prepared according to a method known in the technical field of pharmaceutical preparation. Besides these preparations, Compound A may be prepared as a pharmaceutical preparation for intraocular implant or a pharmaceutical preparation designed for drug delivery such as microsphere.

The "pharmaceutically acceptable carrier" includes various conventional organic or inorganic carrier substances for pharmaceutical materials, e.g. an excipient, a disintegrant, a binder, a fluidizer, and a lubricant for solid preparations, or a solvent, a solubilizing agent, a suspending agent, a tonicity agent, a buffer, and a soothing agent for liquid preparations. Further, an additive including a preserving agent, an antioxidant agent, a colorant, and a sweetening agent may be used as necessary.

The "excipient" includes lactose, white soft sugar, D-mannitol, D-sorbitol, corn starch, dextrin, microcrystalline cellulose, crystalline cellulose, carmellose, carmellose calcium, sodium carboxymethyl starch, low-substituted hydroxypropyl cellulose, gum arabic, etc.

The "disintegrant" includes carmellose, carmellose calcium, carmellose sodium, sodium carboxymethyl starch, croscarmellose sodium, crospovidone, low-substituted hydroxypropyl cellulose, hydroxypropyl methylcellulose, crystalline cellulose, etc.

The "binder" includes hydroxypropyl cellulose, hydroxypropyl methylcellulose, povidone, crystalline cellulose, white soft sugar, dextrin, starch, gelatin, carmellose sodium, gum arabic, etc.

The "fluidizer" includes light anhydrous silicic acid, magnesium stearate, etc.

The "lubricant" includes magnesium stearate, calcium stearate, talc, etc.

The "solvent" includes purified water, ethanol, propylene glycol, macrogol, sesame oil, corn oil, olive oil, etc.

The "solubilizing agent" includes propylene glycol, D-mannitol, benzyl benzoate, ethanol, triethanolamine, sodium carbonate, sodium citrate, etc.

The "suspending agent" includes benzalkonium chloride, carmellose, hydroxypropyl cellulose, propylene glycol, povidone, methylcellulose, glyceryl monostearate, etc.

The "tonicity agent" includes glucose, D-sorbitol, sodium chloride, D-mannitol, etc.

The "buffer" includes sodium hydrogen phosphate, sodium acetate, sodium carbonate, sodium citrate, etc.

The "soothing agent" includes benzyl alcohol, etc.

The "preserving agent" includes ethyl paraoxybenzoate, chlorobutanol, benzyl alcohol, sodium dehydroacetate, sorbic acid, etc.

The "antioxidant agent" includes sodium sulfite, ascorbic acid, etc.

The "colorant" includes food dye (e.g. Food Red No. 2 or No. 3, Food Yellow No. 4 or No. 5), β-carotene, etc.

The "sweetening agent" includes saccharin sodium, dipotassium glycyrrhizinate, aspartame, etc.

An injection can be prepared by using those selected from a tonicity agent such as sodium chloride, a buffer such as sodium phosphate, a surfactant such as polyoxyethylene sorbitan monooleate, and a thickener such as methylcellulose as necessary.

An eye drop can be prepared by using those selected from a tonicity agent such as sodium chloride or concentrated glycerin, a buffer such as sodium phosphate or sodium acetate, a surfactant such as polyoxyethylene sorbitan monooleate, polyoxyl 40 stearate, or polyoxyethylene hardened castor oil, a stabilizer such as sodium citrate or disodium edetate, and an antiseptic agent such as benzalkonium chloride or paraben as necessary, and its pH may be any value within a range acceptable for ophthalmic preparations, and is usually preferably within the range of 4 to 8.

An eye ointment can be prepared by using a widely used base such as white petrolatum and liquid paraffin.

An insert can be prepared by grinding and mixing a biocompatible polymer such as hydroxypropyl cellulose, hydroxypropyl methylcellulose, carboxyvinyl polymer, and polyacrylic acid with Compound A, and compression-molding

the resultant powder. An excipient, a binder, a stabilizer, or a pH modifier may be used as necessary. A preparation for intraocular implant may be prepared by using a biodegradable polymer such as polylactic acid, polyglycolic acid, and lactic acid-glycolic acid copolymer, or a biocompatible polymer such as hydroxypropyl cellulose.

The pharmaceutical composition for use according to the present invention can be orally or parenterally (including rectal administration, intravenous administration, and ophthalmic local administration such as instillation, application of eye ointment and intravitreal injection) administered to a mammal other than a human being (including a mouse, a rat, a hamster, a guinea pig, a rabbit, a cat, a dog, a pig, a cow, a horse, sheep, and a monkey) as well as to a human being. A dosage amount depends on subjects administered, diseases, symptoms, dosage forms, administration routes, etc., and for example, in the case of oral administration to an adult patient (body weight: about 60 kg), the active ingredient Compound A can be administered in about 1 mg to 1 g per day at a time or in several divided doses. An injection can be administered to an adult patient (body weight: about 60 kg), for example, in an amount of 0.0001 to 2000 mg per day at a time or in several divided doses. An eye drop or an insert that, for example, comprises the active ingredient Compound A at a concentration of usually approximately 0.0001% to 0.1% (w/v) can be administered to an adult patient (body weight: about 60 kg) per day at a time or in several divided doses.

Human VEGF includes isoforms such as human VEGF-$A_{121}$, human VEGF-$A_{145}$, human VEGF-$A_{165}$, human VEGF-$A_{189}$, human VEGF-$A_{206}$, human VEGF-$B_{167}$, human VEGF-$B_{186}$, human VEGF-C, human VEGF-D, human PIGF-1 and human PIGF-2, and a quantitatively and qualitatively major subtype is human VEGF-$A_{165}$. The "VEGF" used herein includes the aforementioned isoforms.

Rat VEGF is shorter than human VEGF by one amino acid. Rat VEGF includes various isoforms as is the case with human VEGF, and a quantitatively and qualitatively major subtype is rat $VEGF_{164}$. The "VEGF" used herein includes the aforementioned isoforms.

The phrase "inhibit the functions of VEGF" refers to inhibiting the functions of VEGF to disappear or attenuate the activity thereof, and refers to inhibiting functions of one or two or more molecules located downstream in the signal cascade of VEGF. The phrase "inhibit the functions of VEGF" preferably refers to "inhibit the functions of human VEGF" . The inhibition of functions or the disappearance or attenuation of the activity is conducted preferably in the situations of human clinical application.

The term "treatment" used herein includes amelioration of a symptom, prevention of an aggravation, maintenance of a remission, prevention of an exacerbation, and prevention of a recurrence. The term "prevention" used herein refers to suppressing occurrence of a symptom.

Because Compound A or a pharmaceutically acceptable salt thereof suppresses an increase of the retinal vascular permeability induced by VEGF, it can be used as an active ingredient of a therapeutic or preventive agent for eye diseases involving VEGF.

The eye disease involving VEGF is preferably age-related macular degeneration, diabetic retinopathy, macular edema, retinal vein occlusion, neovascular maculopathy, neovascular glaucoma, etc.

The macular edema is preferably diabetic macular edema or macular edema following branch retinal vein occlusion.

The age-related macular degeneration is preferably exudative age-related macular degeneration. The age-related macular degeneration is more preferably subfoveal choroidal neovascularization secondary to age-related macular degeneration.

The retinal vein occlusion is preferably central retinal vein occlusion.

The present invention provides means for treating or preventing an eye disease selected from the group consisting of age-related macular degeneration, diabetic retinopathy, macular edema, retinal vein occlusion, neovascular maculopathy and neovascular glaucoma, comprising administering to a mammal a therapeutically effective amount of Compound A or a pharmaceutically acceptable salt thereof.

One embodiment of the present invention includes a pharmaceutical composition for use in treating an eye disease selected from the group consisting of age-related macular degeneration, diabetic retinopathy, macular edema, retinal vein occlusion, neovascular maculopathy and neovascular glaucoma, comprising Compound A or a pharmaceutically acceptable salt thereof, and a pharmaceutically acceptable carrier.

The present invention provides means for inhibiting the functions of VEGF comprising administering to a mammal a therapeutically effective amount of Compound A or a pharmaceutically acceptable salt thereof.

The present invention provides means for suppressing an increase of the retinal vascular permeability induced by VEGF comprising administering to a mammal a therapeutically effective amount of Compound A or a pharmaceutically acceptable salt thereof.

The mammal is a mouse, a rat, a hamster, a guinea pig, a rabbit, a cat, a dog, a pig, a cow, a horse, sheep, a monkey, etc., and is preferably a human being.

One embodiment of the present invention includes the Compound A or a pharmaceutically acceptable salt thereof use in treating or preventing an eye disease selected from the group consisting of age-related macular degeneration, diabetic retinopathy, macular edema, retinal vein occlusion, neovascular maculopathy and neovascular glaucoma.

The Compound A or a pharmaceutically acceptable salt thereof for use according to the invention inhibits the functions of VEGF.

The Compound A or a pharmaceutically acceptable salt thereof for use according to the invention suppresses an increase of the retinal vascular permeability induced by VEGF.

The present invention preferably uses a pharmaceutical composition comprising Compound A or a pharmaceutically acceptable salt thereof, and a pharmaceutically acceptable carrier.

Example 1

Experiment 1. Retinal vascular permeability suppression by oral administration of Compound A in rat model where VEGF-induced retinal vascular permeability was increased

A rat model where VEGF-induced retinal vascular permeability was increased was used to evaluate the ability of suppressing an increase of the retinal vascular permeability by Compound A. The animal model was prepared with appropriate modifications based on Non-Patent Literature 3, etc.

As experimental animals, male Brown Norway rats aged 8 weeks {CHARLES RIVER LABORATORIES JAPAN, INC.} were used.

Inside the vitreous body of each eye of rats, rat $VEGF_{164}$ {400 ng/eye, R&D Systems, Inc. } was injected <VEGF-administered group>. Inside the vitreous body of each eye of rats in a normal control group, D-PBS (-) {Sigma-Aldrich Corporation} was intravitreally injected. Each group included four rats <eight eyeballs>.

Compound A suspended in a 1% (w/v) methylcellulose aqueous solution was orally administered at 10 mg/kg, 30 mg/kg or 100 mg/kg 1 hour before, and 4 hours and 20 hours after intravitreal injection of VEGF <Compound A-administered group>. 1% (w/v) methylcellulose aqueous solution in the same volume as that in the Compound A-administered group was orally administered 1 hour before, and 4 hours and 20 hours after intravitreal injection of VEGF <Base-administered group>.

At 24 hours after intravitreal injection of VEGF, rats were euthanized, and the eyeballs of the rats were enucleated while avoiding contamination with blood. Following the enucleation, each eyeball was slightly incised in the vicinity of the optic papilla with a surgical knife, and the vitreous body was quickly enucleated. The protein concentration in the sampled vitreous body was determined by the Bradford method.

The rate of suppressing an increase of the retinal vascular permeability was calculated according to the following equation X.

[Equation X]

$$\text{Rate of suppressing increase of retinal vascular permeability (\%)} = (P_B - P_X) / (P_B - P_A) \times 100$$

$P_A$: Protein concentration in vitreous body of normal control group

$P_B$: Protein concentration in vitreous body of base-administered group

$P_X$: Protein concentration in vitreous body of Compound A-administered group

The evaluation results of Compound A were shown in Fig. 1.

Example 2

Experiment 2. Retinal vascular permeability suppression by intravitreal injection of Compound A in rat model where VEGF-induced retinal vascular permeability was increased

As experimental animals, male Brown Norway rats aged 8 weeks {CHARLES RIVER LABORATORIES JAPAN, INC.} were used.

A mixture of rat $VEGF_{164}$ {400 ng/eye, R&D Systems, Inc.} and Compound A (10 μg/eye, 30 μg/eye or 100 μg/eye) dissolved or suspended in a base was injected into the vitreous body of each eye of rats <Compound A-administered group>. In the vitreous body of each eye of rats of a base-administered group, a mixture of rat $VEGF_{164}$ and the base of the same volume as that of the Compound A-administered group was injected. As the base, an aqueous base was used. In the vitreous body of each eye of rats of a normal control group, D-PBS (-) {Sigma-Aldrich Corporation} was injected. Each group included four rats <eight eyeballs>.

At 24 hours after intravitreal injection of VEGF, rats were euthanized, and the eyeballs of the rats were enucleated while avoiding contamination with blood. Following the enucleation, each eyeball was slightly incised in the vicinity of the optic papilla with a surgical knife, and the vitreous body was quickly enucleated. The protein concentration in the sampled vitreous body was determined by the Bradford method. The rate of suppressing an increase of the retinal vascular permeability was calculated according to the following equation X.

[Equation X]

$$\text{Rate of suppressing increase of retinal vascular permeability (\%)} = (P_B - P_X) / (P_B - P_A) \times 100$$

$P_A$: Protein concentration in vitreous body of normal control group
$P_B$: Protein concentration in vitreous body of base-administered group
$P_X$: Protein concentration in vitreous body of Compound A-administered group

The evaluation results of Compound A were shown in Fig. 2.

Example 3

Experiment 3. Ocular tissue distribution and systematic exposure of Compound A orally administered or intravitreally injected

As experimental animals, male Crl:CD <SD> rats aged 8 weeks {CHARLES RIVER LABORATORIES JAPAN, INC.} were used.

Compound A suspended in a 1% (w/v) methylcellulose aqueous solution was orally administered to rats at 100 mg/kg. At 0.5, 1, 2, 4, 6 and 24 hours after oral administration, plasma and neural retina were sampled, and the concentrations of Compound A in the plasma and in the neural retina were quantified by liquid chromatography mass spectrometry <LC-MS>.

In the vitreous body of each eye of rats, Compound A dissolved or suspended in a base was injected at 10 μg/eye. At 1, 2, 4 and 24 hours after intravitreal injection, neural retina was sampled, and the concentration of Compound A in the neural retina was quantified by LC-MS. Each group included two to three rats <four to six eyeballs>.

Transition of plasma concentration of Compound A orally administered (100 mg/kg) was shown in Table 1, transition of neural retina concentration of Compound A orally administered (100 mg/kg) was shown in Table 2, and transition of neural retina concentration of Compound A intravitreally injected (10 μg/eye) was shown in Table 3.

[Table 1]

| Dose | Plasma concentration of Compound A (μg/mL) orally administered to rats in a single application | | | | | |
|---|---|---|---|---|---|---|
| | Time (hr) | 0.5 | 1 | 2 | 4 | 6 | 24 |
| 100 mg/kg | | 5.81 | 7.95 | 6.52 | 4.86 | 3.04 | 0.03 |
| | | 6.83 | 8.57 | 6.18 | 3.41 | 5.06 | 0.02 |
| | Average | 6.32 | 8.26 | 6.35 | 4.14 | 4.05 | 0.02 |

[Table 2]

| Dose | Neural retina concentration of Compound A (μg/mL) orally administered to rats in a single application | | | | | | |
|---|---|---|---|---|---|---|---|
| | Time (hr) | 0.5 | 1 | 2 | 4 | 6 | 24 |
| 100 mg/kg | | 4.89 | 3.24 | 3.38 | 2.29 | 1.05 | BLQ |
| | | 2.70 | 3.80 | - a) | 2.43 | 1.47 | BLQ |
| | | 2.96 | 3.93 | 2.66 | 2.30 | 2.90 | BLQ |
| | | 4.42 | 4.48 | 2.12 | 2.54 | 2.24 | - a) |
| | Average | 3.74 | 3.86 | 2.72 | 2.39 | 1.92 | NC |

(continued)

| Dose | Neural retina concentration of Compound A (μg/mL) orally administered to rats in a single application | | | | | | |
|---|---|---|---|---|---|---|---|
| | Time (hr) | 0.5 | 1 | 2 | 4 | 6 | 24 |
| | Standard deviation | 1.08 | 0.51 | 0.63 | 0.12 | 0.82 | NC |

a) These samples were excluded because of the mistake of sampling.
BLQ: Below the lower limit of quantification (ca. 0.16 μg/g)
NC: Not calculated

[Table 3]

| Dose | Neural retina concentration of Compound A (μg/mL) intravitreally injected to rats | | | | |
|---|---|---|---|---|---|
| | Time (hr) | 1 | 2 | 4 | 24 |
| 10 μg/eye | | - a) | 4.73 | 0.27 | BLQ |
| | | 52.66 | 12.71 | 1.42 | BLQ |
| | | 45.53 | 15.78 | 1.66 | BLQ |
| | | 42.17 | 5.13 | 1.26 | BLQ |
| | | - a) | 6.28 | 2.07 | BLQ |
| | | 39.49 | 13.49 | 2.64 | BLQ |
| | Average | 44.96 | 9.69 | 1.55 | NC |
| | Standard deviation | 5.70 | 4.85 | 0.80 | NC |

a) These samples were excluded because of the mistake of sampling.
BLQ: Below the lower limit of quantification (ca. 0.16 μg/g)
NC: Not calculated

INDUSTRIAL APPLICABILITY

The present invention provides novel medical use of Compound A for eye diseases as the target disease.

## Claims

1. A compound represented by the following chemical structural formula:

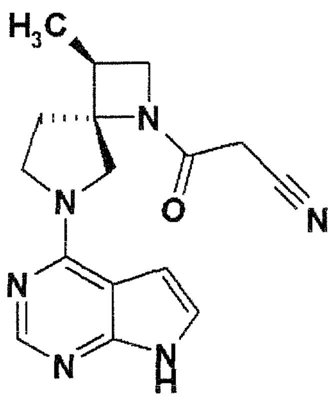

or a pharmaceutically acceptable salt thereof for use in the treatment or prevention of an eye disease selected from the group consisting of age-related macular degeneration, diabetic retinopathy, macular edema, retinal vein occlusion, neovascular maculopathy and neovascular glaucoma.

2. The compound for use according to claim 1, wherein the age-related macular degeneration is exudative age-related

macular degeneration.

3. The compound for use according to claim 1, wherein the macular edema is diabetic macular edema or macular edema following branch retinal vein occlusion.

4. The compound for use according to claim 1, wherein the retinal vein occlusion is central retinal vein occlusion.

5. The compound for use according to claim 1, wherein the compound is to be orally administered.

6. The compound for use according to claim 1, wherein the compound is to be administered to the eyes.

7. The compound for use according to claim 6, wherein an administration site is a vitreous body.

8. The compound for use according to claim 6 or 7, wherein a dosage form is an injection.

9. A pharmaceutical composition for use in the treatment or prevention of an eye disease selected from the group consisting of age-related macular degeneration, diabetic retinopathy, macular edema, retinal vein occlusion, neovascular maculopathy and neovascular glaucoma, comprising as an active ingredient, a compound represented by the following chemical structural formula:

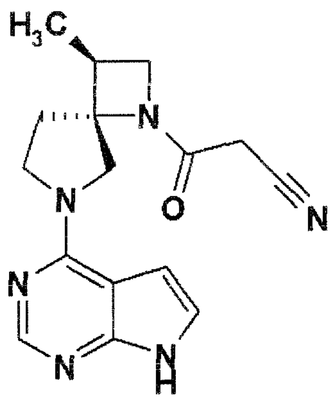

or a pharmaceutically acceptable salt thereof.

10. The pharmaceutical composition for use according to claim 9, wherein the age-related macular degeneration is exudative age-related macular degeneration.

11. The pharmaceutical composition for use according to claim 9, wherein the macular edema is diabetic macular edema or macular edema following branch retinal vein occlusion.

12. The pharmaceutical composition for use according to claim 9, wherein the retinal vein occlusion is central retinal vein occlusion.

13. The pharmaceutical composition for use according to claim 9, wherein the pharmaceutical composition is to be orally administered.

14. The pharmaceutical composition for use according to claim 9, wherein the pharmaceutical composition is to be administered to the eyes.

15. The pharmaceutical composition for use according to claim 14, wherein an administration site is a vitreous body.

16. The pharmaceutical composition for use according to claim 14 or 15, wherein a dosage form is an injection.

**Patentansprüche**

1. Eine Verbindung, dargestellt durch die folgende chemische Strukturformel:

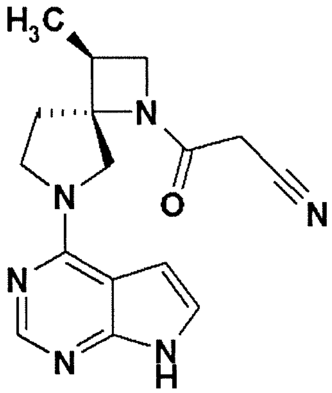

oder ein pharmazeutisch verträgliches Salz davon zur Verwendung bei der Behandlung oder Vorbeugung einer Augenerkrankung, ausgewählt aus der Gruppe bestehend aus altersbedingter Makuladegeneration, diabetischer Retinopathie, Makulaödem, retinalem Venenverschluss, neovaskulärer Makulopathie und neovaskulärem Glaukom.

2. Die Verbindung zur Verwendung nach Anspruch 1, wobei es sich bei der altersbedingten Makuladegeneration um eine feuchte altersbedingte Makuladegeneration handelt.

3. Die Verbindung zur Verwendung nach Anspruch 1, wobei es sich bei dem Makulaödem um diabetisches Makulaödem oder Makulaödem infolge von retinalem Astvenenverschluss handelt.

4. Die Verbindung zur Verwendung nach Anspruch 1, wobei es sich bei dem retinalen Venenverschluss um retinalen Zentralvenenverschluss handelt.

5. Die Verbindung zur Verwendung nach Anspruch 1, wobei die Verbindung oral zu verabreichen ist.

6. Die Verbindung zur Verwendung nach Anspruch 1, wobei die Verbindung an den Augen zu verabreichen ist.

7. Die Verbindung zur Verwendung nach Anspruch 6, wobei ein Verabreichungsort ein Glaskörper ist.

8. Die Verbindung zur Verwendung nach Anspruch 6 oder 7, wobei eine Darreichungsform eine Injektion ist.

9. Ein Arzneimittel zur Verwendung bei der Behandlung oder Vorbeugung einer Augenerkrankung, ausgewählt aus der Gruppe bestehend aus altersbedingter Makuladegeneration, diabetischer Retinopathie, Makulaödem, retinalem Venenverschluss, neovaskulärer Makulopathie und neovaskulärem Glaukom, umfassend als einen Wirkstoff eine Verbindung, dargestellt durch die folgende chemische Strukturformel:

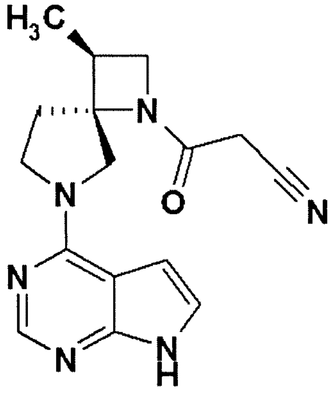

oder ein pharmazeutisch verträgliches Salz davon.

**10.** Das Arzneimittel zur Verwendung nach Anspruch 9, wobei es sich bei der altersbedingten Makuladegeneration um feuchte altersbedingte Makuladegeneration handelt.

**11.** Das Arzneimittel zur Verwendung nach Anspruch 9, wobei es sich bei dem Makulaödem um diabetisches Makulaödem oder Makulaödem infolge von retinalem Astvenenverschluss handelt.

**12.** Das Arzneimittel zur Verwendung nach Anspruch 9, wobei es sich bei dem retinalen Venenverschluss um retinalen Zetralvenenverschluss handelt.

**13.** Das Arzneimittel zur Verwendung nach Anspruch 9, wobei das Arzneimittel oral zu verabreichen ist.

**14.** Das Arzneimittel zur Verwendung nach Anspruch 9, wobei das Arzneimittel an den Augen zu verabreichen ist.

**15.** Das Arzneimittel zur Verwendung nach Anspruch 14, wobei ein Verabreichungsort ein Glaskörper ist.

**16.** Das Arzneimittel zur Verwendung nach Anspruch 14 oder 15, wobei eine Darreichungsform eine Injektion ist.

## Revendications

**1.** Composé représenté par la formule structurale chimique suivante :

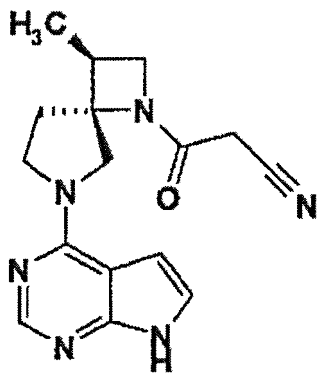

ou l'un de ses sels pharmaceutiquement acceptables pour une utilisation dans le traitement ou la prévention d'une maladie oculaire choisie dans le groupe constitué de la dégénérescence maculaire liée à l'âge, la rétinopathie diabétique, l'oedème maculaire, l'occlusion veineuse rétinienne, la maculopathie néovasculaire et le glaucome néovasculaire.

**2.** Composé pour une utilisation selon la revendication 1, dans lequel la dégénérescence maculaire liée à l'âge est la dégénérescence maculaire liée à l'âge exsudative.

**3.** Composé pour une utilisation selon la revendication 1, dans lequel l'oedème maculaire est l'oedème maculaire diabétique ou l'oedème maculaire suite à une occlusion d'une branche veineuse rétinienne.

**4.** Composé pour une utilisation selon la revendication 1, dans lequel l'occlusion veineuse rétinienne est l'occlusion de la veine centrale de la rétine.

**5.** Composé pour une utilisation selon la revendication 1, dans lequel le composé doit être administré par voie orale.

**6.** Composé pour une utilisation selon la revendication 1, dans lequel le composé doit être administré dans les yeux.

**7.** Composé pour une utilisation selon la revendication 6, dans lequel un site d'administration est un corps vitré.

**8.** Composé pour une utilisation selon la revendication 6 ou 7, dans lequel une forme galénique est une injection.

**9.** Composition pharmaceutique pour une utilisation dans le traitement ou la prévention d'une maladie oculaire choisie dans le groupe constitué de la dégénérescence maculaire liée à l'âge, la rétinopathie diabétique, l'oedème maculaire, l'occlusion veineuse rétinienne, la maculopathie néovasculaire et le glaucome néovasculaire, comprenant en tant que principe actif, un composé représenté par la formule structurale chimique suivante :

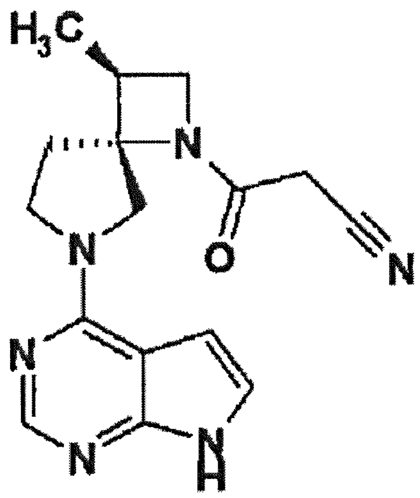

ou l'un de ses sels pharmaceutiquement acceptables.

**10.** Composition pharmaceutique pour une utilisation selon la revendication 9, dans laquelle la dégénérescence maculaire liée à l'âge est la dégénérescence maculaire liée à l'âge exsudative.

**11.** Composition pharmaceutique pour une utilisation selon la revendication 9, dans laquelle l'oedème maculaire est l'oedème maculaire diabétique ou l'oedème maculaire suite à une occlusion d'une branche veineuse rétinienne.

**12.** Composition pharmaceutique pour une utilisation selon la revendication 9, dans laquelle l'occlusion veineuse rétinienne est l'occlusion de la veine centrale de la rétine.

**13.** Composition pharmaceutique pour une utilisation selon la revendication 9, dans laquelle la composition pharmaceutique doit être administrée par voie orale.

**14.** Composition pharmaceutique pour une utilisation selon la revendication 9, dans laquelle la composition pharmaceutique doit être administrée dans les yeux.

**15.** Composition pharmaceutique pour une utilisation selon la revendication 14, dans laquelle un site d'administration est un corps vitré.

**16.** Composition pharmaceutique pour une utilisation selon la revendication 14 ou 15, dans laquelle une forme galénique est une injection.

[Fig. 1]

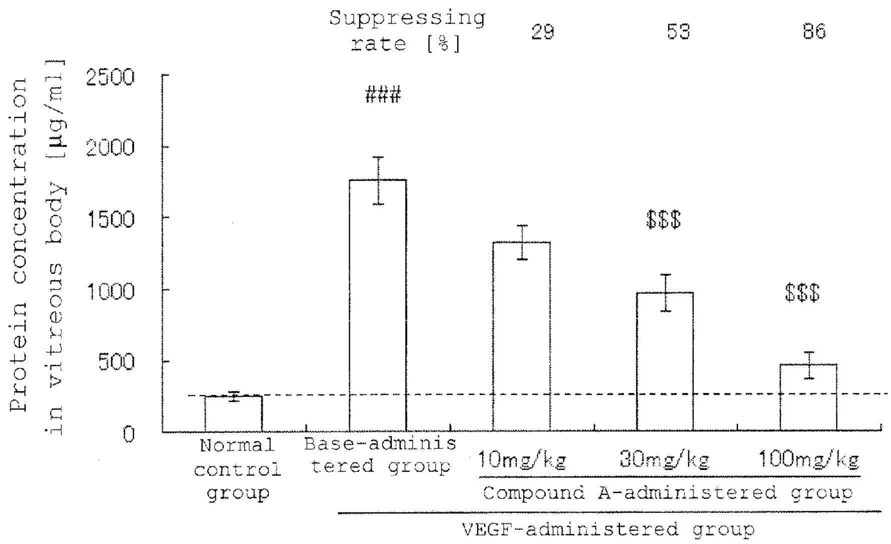


Each column represents the mean ± S.E. (n=8)
###: p<0.001 versus Normal by Aspin-Welch's t-test.
$$$: p<0.001 versus Vehicle by Dunnett's type multiple comparison test.

[Fig. 2]

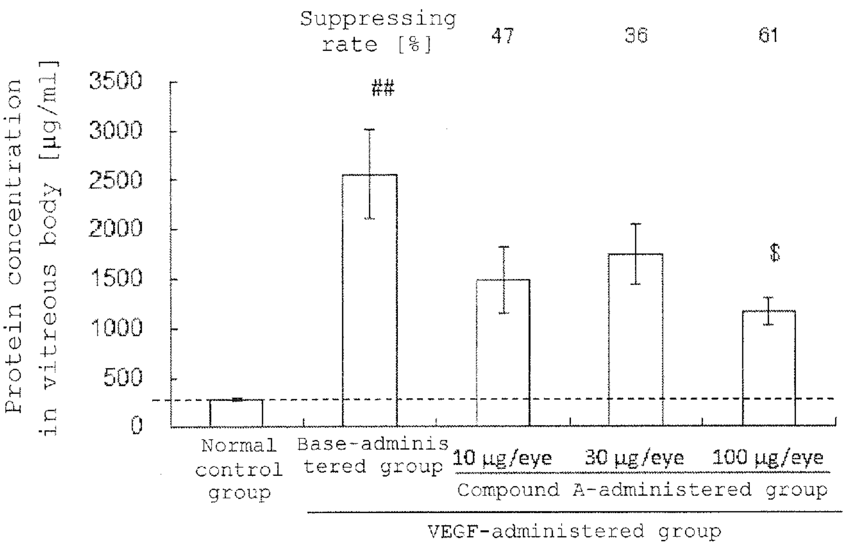


Each column represents the mean ± S.E. (n=7-8)
##: p<0.01 versus Normal by Aspin-Welch's t-test.
$: p<0.05 versus Vehicle by Dunnett's type multiple comparison test.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2011046700 A


### Non-patent literature cited in the description


- **CRAWFORD, Y et al.** VEGF inhibition: insights from preclinical and clinical studies. *Cell Tissue Res.,* January 2009, vol. 335 (1), 261-269
- **DVORAK, HF et al.** Vascular permeability factor/vascular endothelial growth factor and the significance of microvascular hyperpermeability in angiogenesis. *Curr Top Microbiol Immunol,* 1999, vol. 237, 97-132
- **XU, Q et al.** Sensitive blood-retinal barrier breakdown quantitation using Evans blue. *Invest Ophthalmol Vis Sci,* March 2001, vol. 42 (3), 789-794
- **EDELMAN, JL et al.** Corticosteroids inhibit VEGF-induced vascular leakage in a rabbit model of blood-retinal and blood-aqueous barrier breakdown. *Exp Eye Res,* February 2005, vol. 80 (2), 249-258